# EUROPÄISCHE PATENTSCHRIFT

(11) **EP 2 523 968 B1**
(45) Veröffentlichungstag und Bekanntmachung des Hinweises auf die Patenterteilung: **10.09.2014**
(21) Anmeldenummer: 11705798.4
(22) Anmeldetag: 12.01.2011
(51) Int. Cl.: C07K 7/56, C07K 14/525, A61K 38/04

(54) **ZYKLISCHE PEPTIDE ZUR REGULIERUNG VON VEKTORIELLEN IONENKANALEN**
CYCLIC PEPTIDES FOR REGULATION OF VECTORIAL ION CHANNELS
PEPTIDES CYCLIQUES OUR LA RÉGULATION DES CANAUX IONIQUES VECTORIELS

(30) Priorität: 14.01.2010 AT 412010
(43) Veröffentlichungstag der Anmeldung: 21.11.2012
(73) Patentinhaber: Apeptico Forschung und Entwicklung GmbH, 1150 Wien (AT)
(72) Erfinder: FISCHER, Bernhard, A-1160 Wien (AT); LUCAS,Rudolf, B-2630 Aartselaar (BE); TZOTZOS, Susan, A-1180 Wien (AT)
(74) Vertreter: Schwarz, Albin
(86) Internationale Anmeldenummer: PCT/AT2011/000014
(87) Internationale Veröffentlichungsnummer: WO 2011/085423

(56) Entgegenhaltungen:
- EP-A1- 2 009 023
- WO-A1-2009/073909
- WO-A1-2010/099556
- P HAZEMI ET AL.: "Essential structural features of TNF-alpha lectin-like domain derived peptides for activation of amiloride-sensitive sodium currecnt in A549 cells", JOURNAL OF MEDICINAL CHEMISTRY, Bd. 53, Nr. 22, 27. Oktober 2010 (2010-10-27), Seiten 8021-8029, XP002638326, American Chemical Society ISSN: 0022-2623

### SEQUENCE LISTING

<110> APEPTICO Forschung und Entwicklung GmbH
<120> organische verbindungen zur Regulierung von vektoriellen Ionenkanälen
<130> A 14832
<140> A 41/2010
   <141> 2010-01-14
<160> 9
<170> PatentIn version 3.3
<210> 1
   <211> 17
   <212> PRT
   <213> Artificial
<220>
   <223> cyclo Kepsilon1-G17
<220>
   <221> TURN
   <222> (1)..(17)
   <223> Ringschluss zwischen der N-terminalen Aminosäure Lysin (K) und der C-terminalen Aminosäure Glycin (G) mittels einer Amidbindung zwischen dem Stickstoff der epsilon-Aminogruppe der seitenkette des Lysins und dem Kohlenstoff der Karboxylgruppe des Glycins
<400> 1
   Lys Gly Gln Arg Glu Thr Pro Glu Gly Ala Glu Ala Lys Pro Trp Tyr
   1 5 10 15
   Gly
<210> 2
   <211> 17
   <212> PRT
   <213> Artificial
<220>
   <223> cyclo orn-delta1-G17
<220>
   <221> TURN
   <222> (1)..(17)
   <223> x = ornithin
   Ringschluss zw. der N-terminalen Aminosäure Orn und der C-terminalen Aminosäure Gly mittels Amidbindung zw. dem Stickstoff der delta-Aminogruppe der Seitenkette des Ornithins und dem Kohlenstoff der Karboxylgruppe des Glycins
<400> 2
   Xaa Gly Gln Arg Glu Thr Pro Glu Gly Ala Glu Ala Lys Pro Trp Tyr
   1 5 10 15
   Gly
<210> 3
   <211> 16
   <212> PRT
   <213> Artificial
<220>
   <223> cyclo 1-17
<220>
   <221> TURN
   <222> (1)..(16)
   <223> Ringschluss zw. N-terminalen Glycin u. C-terminalen Glycin mittels Amidbindungen zw. Aminogruppe d. N-terminalen Glycins und C1-Karboxylgruppe d. 5-Amino-Pentansäure u. zw. 5-Aminogruppe d. 5-Amino-Pentansäure u. carboxylgruppe d. c-terminalen Glycins
<400> 3
   Gly Gln Arg Glu Thr Pro Glu Gly Ala Glu Ala Lys Pro Trp Tyr Gly
   1 5 10 15
<210> 4
   <211> 16
   <212> PRT
   <213> Artificial
<220>
   <223> cyclo 1-17
<220>
   <221> TURN
   <222> (1)..(16)
   <223> Ringschluss zw. C-terminalem Glycin u. N-terminalem Glycin mittels Amidbindungen zw. Aminogruppe d. N-terminalen Glycins u. C1-Karboxylgruppe d. gamma-Aminobuttersäure u. zw. Aminogruppe d. gamma-Aminobuttersäure u. Karboxylgruppe d. C-terminalen Glycins
<400> 4
   Gly Gln Arg Glu Thr Pro Glu Gly Ala Glu Ala Lys Pro Trp Tyr Gly
   1 5 10 15
<210> 5
   <211> 16
   <212> PRT
   <213> Artificial
<220>
   <223> cyclo 1-Dgamma17
<220>
   <221> TURN
   <222> (1)..(16)
   <223> Ringschluss zw. C-terminaler Asparaginsäure u. N-terminalem Glycin mittels Amidbindungen zw. Aminogruppe d. N-terminalen Glycins u. C1-Karboxylgruppe d. gamma-Aminobuttersäure u. zw. Aminogruppe d. Gamma-Aminobuttersäure u. Kohlenstoff der Karboxylgruppe der C-terminalen Asparaginsäure-seitenkette
<400> 5
   Gly Gln Arg Glu Thr Pro Glu Gly Ala Glu Ala Lys Pro Trp Tyr Asp
   1 5 10 15
<210> 6
   <211> 16
   <212> PRT
   <213> Artificial
<220>
   <223> cyclo 1-Edelta17
<220>
   <221> TURN
   <222> (1)..(16)
   <223> Ringschluss zw. C-terminaler Glutaminsäure u. N-terminalem Glycin mittels Amidbindungen zw. Aminogruppe d. N-terminalen Glycins u. C1-Karboxylgruppe d. 3-Amino-Propansäure u. zw. Aminogruppe d. 3-Amino-Propansäure u. der Glutamin-Seitenkettenkarboxylgruppe
<400> 6
   Gly Gln Arg Glu Thr Pro Glu Gly Ala Glu Ala Lys Pro Trp Tyr Glu
   1 5 10 15
<210> 7
   <211> 15
   <212> PRT
   <213> Artificial
<220>
   <223> cyclo 1-16
<220>
   <221> TURN
   <222> (1)..(15)
   <223> Ringschluss zw. C-terminalem Tyrosin u. N-terminalem Glycin mittels Amidbindungen zw. Aminogruppe d. N-terminalen Glycins u. C1-Karboxylgruppe d. 7-Amino-Heptansäure u. zw. Aminogruppe d. 7-Amino-Heptansäure u. Tyrosin-Karboxylgruppe
<400> 7
   Gly Gln Arg Glu Thr Pro Glu Gly Ala Glu Ala Lys Pro Trp Tyr
   1 5 10 15
<210> 8
   <211> 16
   <212> PRT
   <213> Artificial
<220>
   <223> cyclo 1-17
<220>
   <221> TURN
   <222> (1)..(16)
   <223> Ringschluss zw. C-terminalem Glycin u. N-terminalem Glycin mittels Amidbindungen zw. Aminogruppe d. N-terminalen Glycins u. C1-Karboxylgruppe d. 6-Amino-Hexansäure u. zw. Aminogruppe d.
<400> 8
   Gly Gln Arg Glu Thr Pro Glu Gly Ala Glu Ala Lys Pro Trp Tyr Gly
   1 5 10 15
<210> 9
   <211> 15
   <212> PRT
   <213> Artificial
<220>
   <223> Grundstrukturelement der erfindungsgemäßen zyklischen organischen Verbindungen
<400> 9
   Gly Gln Arg Glu Thr Pro Glu Gly Ala Glu Ala Lys Pro Trp Tyr
   1 5 10 15

## Beschreibung

Die Erfindung betrifft organische Verbindungen und deren pharmazeutischen Zubereitungen, welche zur Regulierung vektorieller Ionenkanäle, von Krankheiten die mit der Lungenfunktion assoziiert sind und zur Behandlung von Ödemen geeignet sind.

### Stand der Technik

Der Flüssigkeitstransport durch Zellschichten und Gewebe beruht primär auf einem osmotischen Gradienten durch einen vektoriellen Ionentransport, z. B. Natriumtransport. Dieser wird hauptsächlich durch streng regulierten und vital bedeutenden Ionenkanäle, wie z. B. dem epithelialen Natrium-Kanal-Komplex (ENaC), bewerkstelligt (Ware L.B. and Matthay M.A. New England J Med 2001; 342/18: 1334-1359. Matthay et al., Am J Physiol 1996; 270:L487-L503; Berthiaume Y. and Matthay M.A. Respiratory Physiology & Neurobiology 159 (2007) 350-359). Wasser folgt diesem Gradienten passiv, unter anderem durch spezielle Wasserkanäle, wie dem Wasserkanal Aquaporin V. Daher ergäbe sich aus einer medikamentösen Regelung des vektoriellen Ionentransportes durch Zellen und Gewebe die Möglichkeit, den Flüssigkeitsgehalt von Geweben zu regulieren, sowie solche Krankheiten präventiv oder therapeutisch zu behandeln, welche mit einer Ansammlung von Flüssigkeit im Gewebe verbunden sind.

Spricht man von einem Ödem, so ist damit eine pathologische Flüssigkeitsansammlung in einem Organ, wie z. B. der Lunge, aber auch dem Gehirn oder der Haut, gemeint. Bei einem Ödem der Lunge spricht man von einem Lungenödem. Das Lungenödem basiert meist auf dem Ungleichgewicht zwischen Flüssigkeitsextravasation und Flüssigkeitsrückresorption. Sehr oft ist auch die Permeabilität des Lungengewebes geschädigt, sodass eine vermehrte Flüssigkeitszufuhr stattfindet, und sich die Flüssigkeit in den Lungenblässchen (Alveolen) ansammelt.

Das Lungenödem als Folge eines fehlenden Rücktransportes von Flüssigkeit aus den Lungenblässchen in das Interstitium ist besonders bedeutsam bei der Akuten Lungen Verletzung (englisch Acute Lung Injury, ALI), beim Akuten Respiratorischem Distress Syndrom (englisch Acute Respiratory Distress Syndrome, ARDS), beim schweren akuten Atemnotsyndrom (SARS), bei Pneumonie, bei Influenza und anderen bakteriell und viral bedingten Lungenkrankheiten. Das Lungenödem spielt aber auch eine bedeutende Rolle bei anderen Lungenerkrankungen, wie den Beatmung-induzierten Lungenschäden, Lungentransplantationen, Transfusion-assoziierten Lungenschäden, therapeutischer Gabe von IL-2 oder Asthma.

Als Ergebnis einer erhöhten Flüssigkeitsansammlung im Gewebe oder Organ, z. B. der Lunge, wird der notwendige Gasaustausch behindert oder völlig eingeschränkt. Es kommt kein Sauerstoff aus der Atemluft in das Blut, sodass durch Sauerstoffmangel lebensgefährliche Organschäden auftreten können.

Lucas et al (Lucas R et al. Science 1994, 263: 814) beschreibt ein, von der Region Ser(99) bis Glu(116) des Tumor Nekrose Faktors abgeleitetes Peptid, welche den Flüssigkeitsgehalt der Lungenbläschen regulieren sollen.

Dieses Peptid mit der Sequenzen CGQRETPEGAEKPWYC ist auch Gegenstand von WO00/09149.

Ein Peptid ebenfalls zur Regulation des Flüssigkeitsgehaltes der Alveolen mit der Sequenz CGTKPIELGPDEPKAVC ist in EP 2 009 023, und ein Peptid mit der Sequenz LSPGQRETPEGAEAKPWYE ist in WO2009/073909 enthalten.

Bisher gibt es keine gezielte und medizinisch verwendbare Therapie oder Behandlung zur Regulation vektorieller Ionenkanäle in Zellen und Geweben, insbesondere zur Regulation vektorieller lonenkanäle des Lungengewebes. Bisher gibt es auch keine gezielte Therapie zur Regulierung des vektoriellen Ionentransportes der Lunge und insbesondere zur Behandlung des Lungenödems. Ganz allgemein bemüht man sich, Patienten mit einem Lungenödem künstlich zu beatmen, um die Zufuhr von Sauerstoff ins Blut und somit zu den Organen zu gewährleisten.

Der vorliegenden Erfindung liegt somit die Aufgabe zugrunde, organische und bio-aktive Substanzen zur Verfügung zu stellen, welche zur vektoriellen Aktivierung von Ionenkanälen geeignet sind. Insbesondere ist es Ziel der vorliegenden Erfindung, solche organischen und bio-aktive Substanzen zur Verfügung zu stellen, welche zur Aktivierung von epithelialen Natrium-Ionenkanälen der Lunge und zur gezielten Behandlung des Lungenödems verwendet werden können.

Überraschenderweise wurden nun organische Verbindungen gefunden, die zur Lösung der gestellten Aufgabe geeignet sind.

In einem Aspekt stellt die vorliegende Erfindung eine zyklische organische Verbindung zur Verfügiung, die die Amino äuresequenz GQRETPEGAEAKPWY neben einer oder zwei weiteren Aminosäuren umfasst, und die dadurch gekennzeichnet ist, dass sie C-terminal über keine Karboxylgruppe und/oder N-terminal über keine Aminogruppe verfügt, wobei eine der Aminosäuren eine nicht natürliche Aminosäure ist, ausgewählt aus Ornithin oder einer omega-Aminosäure, und wobei der Ringschluss zwischen einer Seitenkette einer Aminosäure mit dem C-Terminus einer anderen Aminosäure ausgebildet ist, oder der Ringschluss ist erfolgt mit Hilfe einer nicht natürlichen Aminosäure; oder die zyklische organische Verbindung ist eine Verbindung der SEQ ID NO: 1

Eine zyklische, organische Verbindung, oder zyklische, organische Verbindungen, die gemäß vorliegende Erfindung zur Verfügung gestellt werden, werden in dieser Anmeldung auch als "Verbindung(en) gemäß vorliegender Erfindung" bezeichnet.

Eine Verbindung gemäß vorliegender Erfindung schließt eine Verbindung in jeglicher Form, z. B. in freier Form und in der Form von Co-Kristallen, z.B. in der Form eines Salzes, oder in der Form eines Solvates, oder in der Form eines Salzes und eines Solvates ein.

In einem weiteren Aspekt stellt die vorliegende Erfindung eine Verbindung gemäß vorliegender Erfindung in der Form eines Salzes zur Verfügung.

Solche Salze schließen vorzugsweise pharmazeutisch annehmbare Salze ein, obwohl pharmazeutisch nicht akzeptable Salze eingeschlossen sind, z. B. zum Zwecke der Herstellung, Isolation, Reinigung einer Verbindung der vorliegenden Erfindung. Beispielsweise schließt die vorliegende Erfindung ein Salz einer Verbindung der vorliegenden Erfindung mit Trifluoressigsäure, das beispielsweise bei der Herstellung einer Verbindung der vorliegenden Erfindung auftreten kann, ein.

Eine Verbindung gemäß vorliegender Erfindung in der Form eines Salzes schließt ein Metallsalz oder ein Säureadditionssalz ein. Metallsalze schließen beispielsweise Alkali oder Erdalkalisalze, Säureadditionssalze ein Salz einer Verbindung gemäß vorliegender Erfindung mit einer Säure ein.

Eine Verbindung gemäß vorliegender Erfindung in freier Form, gegebenenfalls in der Form eines Solvates, kann in eine entsprechende Verbindung in der Form eines Salzes, in Nicht-Solvatform oder in der Form eines Solvates, umgewandelt werden und vice versa.

In Verbindungen gemäß vorliegender Erfindung führen überraschenderweise bestimmte Aminosäuresequenzen in Kombination mit bisher bei Peptiden unbekannten Ringschlüssen und unter Bildung von bisher in Peptiden nicht bekannten, innermolekularer Amidbindung zu zyklischen, organischen Verbindungen, wobei solche Verbindungen völlig unerwartet vektorielle Ionenkanäle in Zellen und Geweben regulieren können, z. B. können Verbindungen der vorliegenden Erfindung den epithelialen Natrium-Kanal-Komplex regulieren, und zwar zum Teil in stärkerem Ausmaß als bisher bekannte, aber strukturell verschiedene Peptide.

Es hat sich überraschenderweise herausgestellt, dass eine Verbindung gemäß vorliegender Erfindung die Aminosäuresequenz GQRETPEGAEAKPWY enthält.

Die nicht natürliche Aminosäure in einer Verbindung gemäß vorliegender Erfindung ist bevorzugt ausgewählt aus Ornithin oder einer omega-Aminosäure, insbesondere einer omega-Amino-(C₃₋₈)Alkansäure, insbesondere aus 3-Amino-Propansäure, gamma-Aminobuttersäure, 5-Amino-Pentansäure, 6-Amino-Hexansäure und 7-Amino-Heptansäure, insbesondere ist die nicht natürliche Aminosäure über Amidbindungen verknüpft.

In einem weiteren Aspekt stellt die vorliegende Erfindung eine Verbindung gemäß vorliegender Erfindung zur Verfügung, in der die nicht natürliche Aminosäure ausgewählt ist aus Ornithin oder einer omega-Aminosäure; insbesondere ist die nicht natürliche Aminosäure über Amidbindungen verknüpft.

In einer Verbindung gemäß vorliegender Erfindung ist bevorzugt der Ringschluss zwischen einer Seitenkette einer Aminosäure mit dem C-Terminus einer anderen Aminosäure, insbesondere zwischen einer Seitenkette des Ornithins oder Lysins, mit dem C-Terminus einer natürlichen Aminosäure, insbesondere eines Glycins, ausgebildet.

In einem weiteren Aspekt stellt die vorliegende Erfindung eine Verbindung gemäß vorliegender Erfindung zur Verfügung, die dadurch gekennzeichnet ist, dass der Ringschluss zwischen einer Seitenkette einer Aminosäure mit dem C-Terminus einer anderen Aminosäure ausgebildet ist.

In einem weiteren Aspekt stellt die vorliegende Erfindung eine Verbindung gemäß vorliegender Erfindung mit der Aminosäuresequenz und zur Verfügung.

In einer Verbindung mit der Sequenz SEQ ID NO: 1 {[KGQRETPEGAEAKPWYG] (cyclo Kepsilon1-G17)} sind die Aminosäuren von der C-terminalen Aminosäure Glycin (G) bis zur N-terminalen Aminosäure Lysin (K) peptidisch verknüpft, während die N-terminale Aminosäure Lysin (K) mit der C-terminalen Aminosäure Glycin (G) mit Hilfe einer Amidbindung zwischen dem Stickstoff der epsilon-Aminogruppe der Seitenkette des Lysins und dem Kohlenstoff der Karboxylgruppe des Glycins verbunden ist, sodass die Verbindung über keine C-terminale Karboxylgruppe verfügt.

In einer Verbindung mit der Sequenz SEQ ID NO: 2 {[Ornithin-GQRETPEGAEAKPWYG] (cyclo Orn-delta1-G17)} sind die Aminosäuren von der C-terminalen Aminosäure Glycin (G) bis zur N-terminale Aminosäure Ornithin (Orn) peptidisch verknüpft, während die N-terminale Aminosäure Ornithin (Orn) mit der C-terminalen Aminosäure Glycin (G) mit Hilfe einer Amidbindung zwischen dem Stickstoff der delta-Aminogruppe der Seitenkette des Ornithins und dem Kohlenstoff der Karboxylgruppe des Glycins verbunden ist, sodass die Verbindung über keine C-terminale Karboxylgruppe verfügt.

In einer Verbindung mit der Sequenz SEQ ID NO: 3 {[5-Amino-Pentansäure-GQRETPEGAEAKPWYG] (cyclo 1-17)} sind die Aminosäuren von der C-terminalen Aminosäure Glycin (G) bis zur N-terminalen Aminosäure Glycin (G) peptidisch verknüpft, während die N-terminale Aminosäure Glycin (G) mit der C-terminalen Aminosäure Glycin (G) mit Hilfe einer Amidbindung zwischen dem Stickstoff der Aminogruppe des N-terminalen Glycin und dem Kohlenstoff C1 der Karboxylgruppe der 5-Amino-Pentansäure einerseits, und durch eine Amidbindung zwischen dem Stickstoff der 5-Aminogruppe der 5-Amino-Pentansäure und dem Kohlenstoff der Karboxylgruppe des C-terminalen Glycins andererseits verbunden ist, sodass die Verbindung über keine C-terminale Karboxylgruppe verfügt.

In einer Verbindung mit der Sequenz SEQ ID NO: 4 {[gamma-Aminobuttersäure-GQRETPEGAEAKPWYG] (cyclo 1-17)} sind die Aminosäuren von der C-terminalen Aminosäure Glycin (G) bis zur N-terminalen Aminosäure Glycin (G) peptidisch verknüpft, während die C-terminale Aminosäure Glycin (G) mit der N-terminalen Aminosäure Glycin (G) mit Hilfe einer Amidbindung zwischen dem Stickstoff der Aminogruppe des N-terminalen Glycins mit dem Kohlenstoff C1 der Karboxylgruppe der gamma-Aminobuttersäure einerseits, und mit Hilfe einer Amidbindung zwischen dem Stickstoff der Aminogruppe der gamma-Aminobuttersäure mit dem Kohlenstoff der Karboxylgruppe des C-terminalen Glycins andererseits verbunden ist, sodass die Verbindung über keine C-terminale Karboxylgruppe verfügt.

In einer Verbindung mit der Sequenz SEQ ID NO: 5 {[gamma-Aminobuttersäure-GQRETPEGAEAKPWYD-OH] (cyclo 1- Dγ17)} sind die Aminosäuren von der C-terminalen Asparaginsäure (D) bis zur N-terminalen Aminosäure Glycin peptidisch verknüpft, während die C-terminale Asparaginsäure (D) mit der N-terminalen Aminosäure Glycin mit Hilfe einer Amidbindung zwischen dem Stickstoff der Aminogruppe des N-terminalen Glycin und dem Kohlenstoff C 1 der Karboxylgruppe der gamma-Aminobuttersäure einerseits, und mit Hilfe einer Amidbindung zwischen dem Stickstoff der Aminogruppe der gamma-Aminobuttersäure und dem Kohlenstoff der Karboxylgruppe der Seitenkette der C-terminalen Asparaginsäure andererseits, verbunden ist, sodass die Verbindung über keine N-terminale Aminogruppe verfügt.

In einer Verbindung mit der Sequenz SEQ ID NO: 6 {[3-Amino-Propansäure-GQRETPEGAEAKPWYE-OH] (cyclo 1- Eδ17)} sind die Aminosäuren von der C-terminalen Glutaminsäure (E) bis zur N-terminalen Aminosäure Glycin peptidisch verknüpft, während die C-terminale Glutaminsäure (E) mit der N-terminalen Aminosäure Glycin mit Hilfe einer Amidbindung zwischen dem Stickstoff der Aminogruppe des N-terminalen Glycins und dem Kohlenstoff C1 der Karboxylgruppe der 3-Amino-Propansäure einerseits, und mit Hilfe einer Amidbindung zwischen dem Stickstoff der Aminogruppe der 3-Amino-Propansäure und dem Kohlenstoff der Karboxylgruppe der Seitenkette der C-terminalen Glutaminsäure andererseits verbunden ist, sodass die Verbindung über keine N-terminale Aminogruppe verfügt.

In einer Verbindung mit der Sequenz SEQ ID NO: 7 {[7-Amino-Heptansäure-GQRETPEGAEAKPWY] (cyclo 1-16)} sind die Aminosäuren von der C-terminalen Aminosäure Tyrosin bis zur N-terminalen Aminosäure Glycin peptidisch verknüpft, während die C-terminale Aminosäure Tyrosin mit der N-terminalen Aminosäure Glycin mit Hilfe einer Amidbindung zwischen dem Stickstoff der Aminogruppe des N-terminalen Glycins mit dem Kohlenstoff C1 der Karboxylgruppe der 7-Amino-Heptansäure einerseits, und mit Hilfe einer Amidbindung zwischen dem Stickstoff der Aminogruppe der 7-Amino-Heptansäure und dem Kohlenstoff der Karboxylgruppe des C-terminalen Tyrosins andererseits, verbunden ist, sodass die Verbindung weder über eine N-terminale Aminogruppe, noch über eine C-terminal Karboxylgruppe verfügt.

In einer Verbindung mit der Sequenz SEQ ID NO: 8 {[6-Amino-Hexansäure-GQRETPEGAEAKPWYG] (cyclo 1-17)} sind die Aminosäuren von der C-terminalen Aminosäure Glycin bis zur N-terminalen Aminosäure Glycin peptidisch verknüpft, während die C-terminalen Aminosäure Glycin mit der N-terminalen Aminosäure Glycin mit Hilfe einer Amidbindung zwischen dem Stickstoff der Aminogruppe des N-terminalen Glycins und dem Kohlenstoff C1 der Karboxylgruppe der 6-Amino-Hexansäure einerseits, und mit Hilfe einer Amidbindung zwischen dem Stickstoff der Aminogruppe der 6-Amino-Hexansäüre und dem Kohlenstoff der Karboxylgruppe des C-terminalen Glycins andererseits verbunden ist, sodass die Verbindung weder über eine N-terminale Aminogruppe, noch über eine C-terminal Karboxylgruppe verfügt.

Eine Verbindung gemäß vorliegender Erfindung kann in geeigneter Weise hergestellt werden, z. B. analog zu einem bekannten Verfahren, oder wie hierin beschrieben, beispielsweise durch chemische Synthese oder mittels mikrobieller Verfahren, wobei insbesondere die Einführung einer Amidbindung zwischen einer freien Aminogruppe und einer freien Karboxylgruppe in geeigneter Weise erfolgen kann, z. B. analog zu einem bekannten Verfahren, oder wie in der vorliegenden Anmeldung beschrieben.

Es hat sich gezeigt, dass eine Verbindung gemäß vorliegender Erfindung interessante pharmakologische Aktivität zeigt und daher als Medikament verwendet werden kann.

In einem weiteren Aspekt stellt die vorliegende Erfindung eine Verbindung gemäß vorliegender Erfindung zur Verwendung als ein Medikament zur Verfügung.

Biologische Untersuchungen an humanen Zellen zeigen, dass die Verbindungen gemäß vorliegender Erfindung keine entzündlichen oder toxischen Eigenschaften aufweisen. Dazu werden in laborüblicher Zellkultur humane Epithelzellen kultiviert, und eine Verbindung gemäß vorliegender Erfindung beigefügt. Trotz Zugabe einer Verbindung der vorliegenden Erfindung wurden keine toxischen oder entzündlichen Reaktionen an den humanen Zellen beobachtet.

Der Nachweis einer vektoriellen Regulierung von Ionenkanälen durch eine Verbindung kann gemäß einer laborüblichen Methode, zum Beispiel gemäß Clunes M.T., et al., J. Physiolo. (2004) 557.3: 809-819), mittels Patch-Clamp Experimenten erfolgen. Für Patch-Clamp Untersuchungen an Ionenkanälen wird eine Glaskanüle dünn ausgezogene und mit neutraler Pufferlösung Lösung gefüllt. Die Glaskanüle (Patch-Clamp-Pipette) wird vorsichtig auf eine intakte epitheliale Zelle gedrückt. Unterhalb der Pipette befindet sich ein Stück Membran. Zwischen dem Inneren der Pipette und der Außenlösung entsteht dadurch ein elektrischer Widerstand. In die Pipettenlösung taucht eine Elektrode, die an einen empfindlichen Verstärker angeschlossen ist.

Eine Regulierung der vektoriellen epithelialen Ionenkanäle wird durch die Veränderung der Stromstärke bei konstanter Spannung nachgewiesen.

Auf diese Weise hat sich überraschenderweise herausgestellt, dass die Verbindungen der vorliegenden Erfindung eine Regulierung der vektoriellen epithelialen Ionenkanäle zeigen. Ganz besonders überraschend war, dass Verbindungen gemäß vorliegender Erfindung, z. B. Verbindungen mit der Aminosäuresequenz SEQ ID NO: 1, SEQ ID NO: 2, SEQ ID NO: 5, SEQ ID NO: 6 und SEQ ID NO: 8 zu deutlich höheren Aktivierungen des vektoriellen Ionenstromes führen, als die bereits aus der Literatur bekannten Peptide CGQRETPEGAEKPWYC (Lucas et al. Science 1994, auch WO00/09149), CGTKPIELGPDEPKAVC (SEQ ID No. 2 aus EP 2009 023) und LSPGQRETPEGAEAKPWYE (SEQ ID No. 2. aus PCT AT2008 448).

Eine Verbindung gemäß vorliegender Erfindung kann somit zur Herstellung eines Medikamentes, z. B. zur Regulierung von vektoriellen Ionenkanälen, insbesondere von Ionenkanälender Lunge, und zur Behandlung von Ödemen, insbesondere zur Behandlung des Lungenödems, verwendet werden; und in einem weiteren Aspekt stellt die vorliegende Erfindung eine Verbindung gemäß vorliegender Erfindung zur Herstellung eines Medikamentes zur Regulierung von vektoriellen Ionenkanälen, insbesondere von Ionenkanälen der Lunge, zur Behandlung von Krankheiten die mit der Lungenfunktion assoziiert sind, und zur Behandlung von Ödemen, insbesondere zur Behandlung des Lungenödems, zur Verfügung.

Die Behandlung von Krankheiten die mit der Lungenfunktion assoziiert sind, schließt z. B. die Aktivierung epithelialer Ionenkanäle, die Verbesserung der Lungenfunktion und/oder die Behandlung von Ödemen, wie Lungenödemen,
weiters die Behandlung
- der Akuter Lungen Verletzung (engl. Acute Lung Injury, ALI),
- des Akuten Respiratorischen Distress Syndrom (engl. Acute Respiatory Distress Syndrome, ARDS),
- des schweren akuten Atemnotsyndroms (SARS),
- der Pneumonie,
- viraler Lungenentzündungen, wie Influenza- und RSV-Infektionen;
- bei Multiorganversagen,
- bei Beatmung-induzierter Lungenschäden, Lungentransplantationen, TransfusionassoziierterLungenschäden, therapeutischer Gabe von IL-2 oder Asthma
ein.

In einem anderen Aspekt stellt die vorliegende Erfindung ein Verfahren zur Regulierung von vektoriellen Ionenkanälen, insbesondere von Ionenkanälender Lunge, zur Behandlung von Krankheiten die mit der Lungenfunktion assoziiert sind, und zur Behandlung von Ödemen, insbesondere zur Behandlung des Lungenödems, zur Verfügung, das dadurch gekennzeichnet ist, dass einem Patienten, der einer solchen Behandlung bedarf, eine ausreichende Menge einer Verbindung gemäß vorliegender Erfindung verabreicht wird.

Ein Patient, wie hierin verwendet, schließt Säugetiere, z. B. Menschen, ein.

Eine Verbindung gemäß vorliegender Erfindung kann in der Form einer pharmazeutischen Zubereitung verabreicht werden.

In einem anderen Aspekt stellt die vorliegende Erfindung eine pharmazeutische Zubereitung zur Verfügung, die dadurch gekennzeichnet ist, dass sie eine Verbindung gemäß vorliegender Erfindung umfasst, z. B. in Verbindung mit zumindest einem pharmazeutisch akzeptablen Hilfsstoff, wie Träger oder Verdünnungsmittel, beispielsweise in Verbindung mit einem oder mehreren Füllstoffen, Bindemitteln, Sprengmitteln, Verbesserern der Fließeigenschaften, Gleitmitteln, Geschmacksstoffen, Zuckern oder Süßstoffen, Geruchsstoffen, Konservierungsstoffen, stabilisierend wirkenden Stoffen, Netzmitteln, Emulgatoren, Lösungsvermittlern, Salzen zur Regulierung des osmotischen Drucks und/oder Puffer(mischungen).

Die geeignete Menge Verbindung gemäß vorliegender Erfindung zur Behandlung von Krankheiten wird natürlich sehr von verschiedenen Parametern abhängen, beispielsweise der chemischen Natur und der Pharmakokinetik der verwendeten Verbindung, dem individuellen Patienten, der zu behandelnden Krankheit, der Art der Anwendung; aber eine erfolgreiche tägliche Dosis in größeren Säugetieren schließt beispielsweise eine Menge von 0.0001 g bis 1.5 g ein, z. B. 0.001 mg/kg Körpergewicht bis etwa 20 mg/kg Körpergewicht ein.

Verbindungen gemäß vorliegender Erfindung können in freier Form, oder in der Form eines Salzes, gegebenenfalls in der Form eines Solvates, verabreicht werden. Eine Verbindung gemäß vorliegender Erfindung in der Form eines Salzes, gegebenenfalls in der Form eines Solvates, weist im wesentlichen die gleiche Aktivität auf, wie eine Verbindung der vorliegenden Erfindung in freier, gegebenenfalls nicht-solvatisierter Form, auf.

Die Verabreichung einer Verbindung gemäß vorliegender Erfindung oder deren pharmazeutische Zubereitung kann bevorzugt pulmonal oder parenteral erfolgen und erfolgt besonders bevorzugt pulmonal.

Eine pharmazeutische Zubereitung gemäß vorliegender Erfindung kann in geeigneter Weise hergestellt werden, z. B. analog einer bekannten Methode, z. B. durch Misch-, Granulier-, Beschichtungs-, Lösungs-, Lyophilisierungsverfahren.

### Beschreibung der Abbildungen

Fig. 1 zeigt das HPLC Chromatogramm einer Verbindung mit der Aminosäuresequenz SEQ ID NO: 1.
Fig. 2 zeigt das HPLC Chromatogramm einer Verbindung mit der Aminosäuresequenz SEQ ID NO: 2.
Fig. 3 zeigt das HPLC Chromatogramm einer Verbindung mit der Aminosäuresequenz SEQ ID NO: 3.
Fig. 4 zeigt das HPLC Chromatogramm einer Verbindung mit der Aminosäuresequenz SEQ ID NO: 4.
Fig. 5 zeigt das HPLC Chromatogramm einer Verbindung mit der Aminosäuresequenz SEQ ID NO: 5.
Fig. 6 zeigt das HPLC Chromatogramm einer Verbindung mit der Aminosäuresequenz SEQ ID NO: 6.
Fig. 7 zeigt das HPLC Chromatogramm einer Verbindung mit der Aminosäuresequenz SEQ ID NO: 7.
Fig. 8 zeigt das HPLC Chromatogramm einer Verbindung mit der Aminosäuresequenz SEQ ID NO: 8.

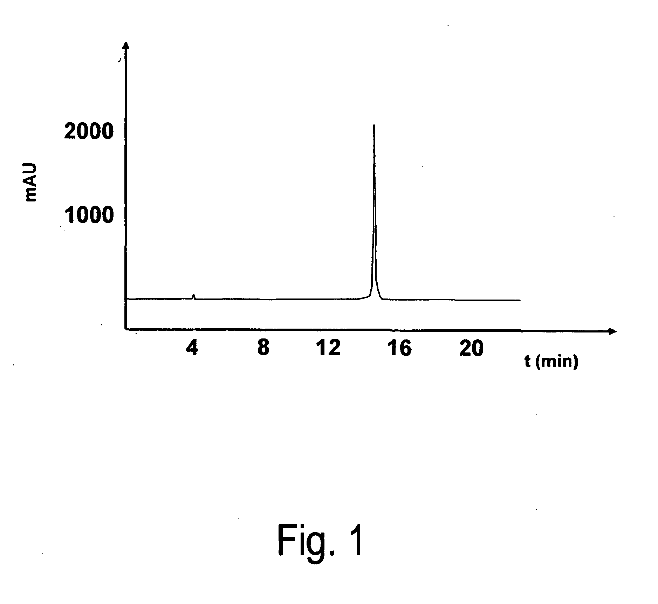

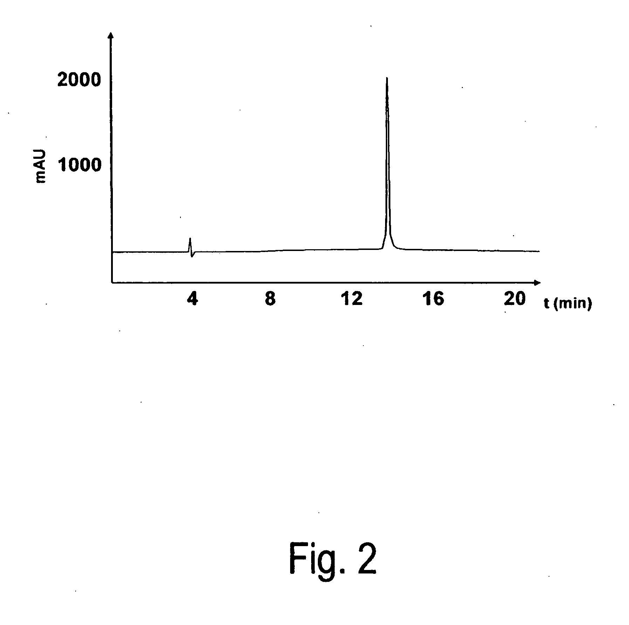

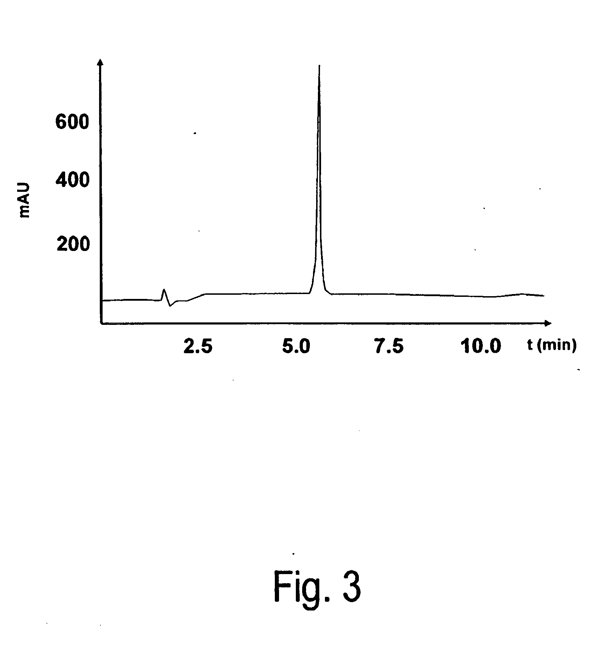

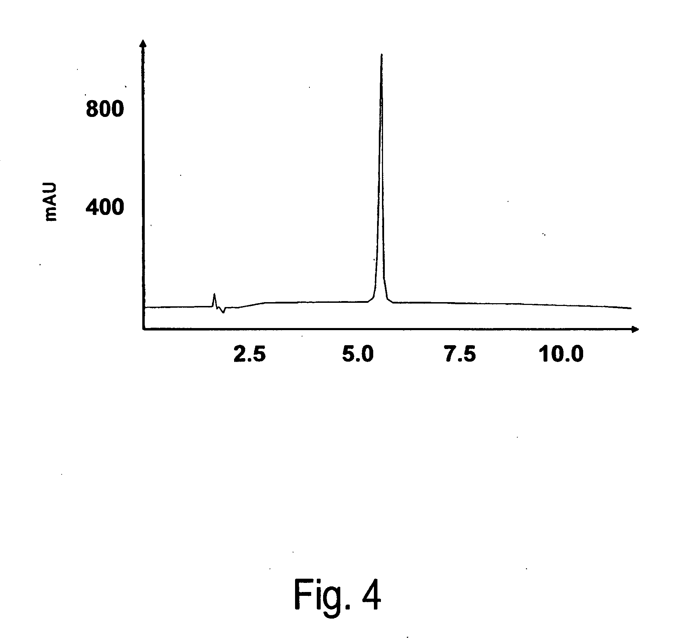

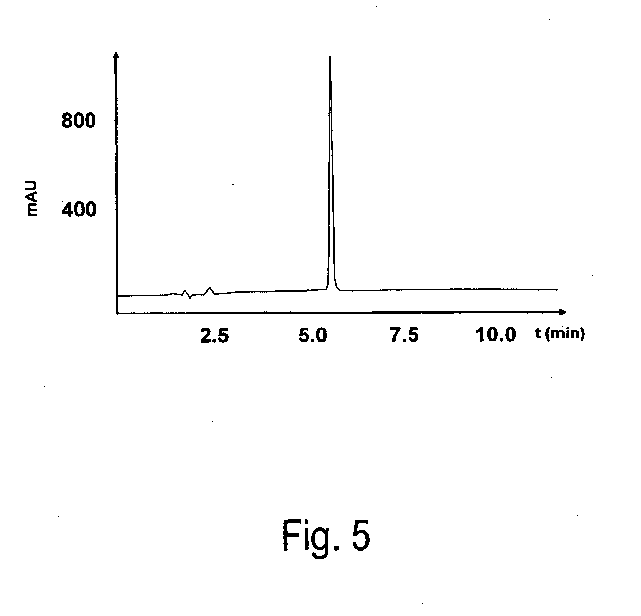

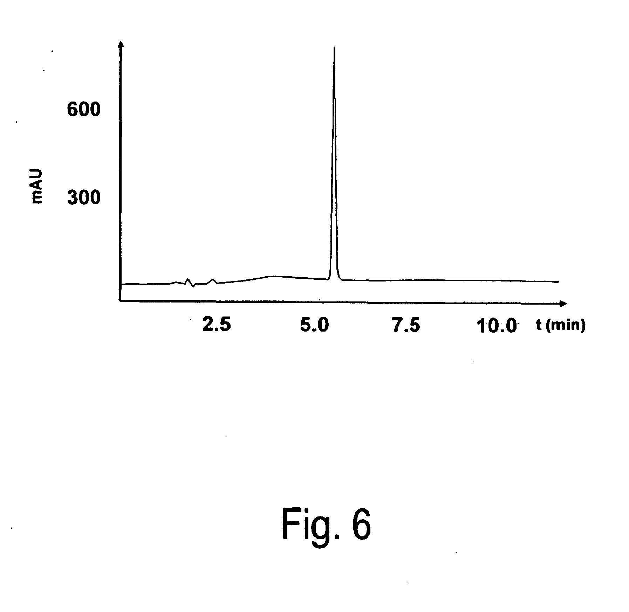

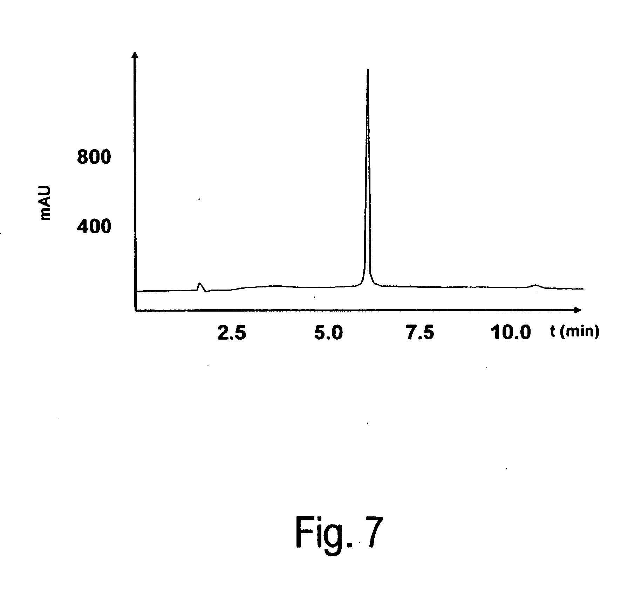

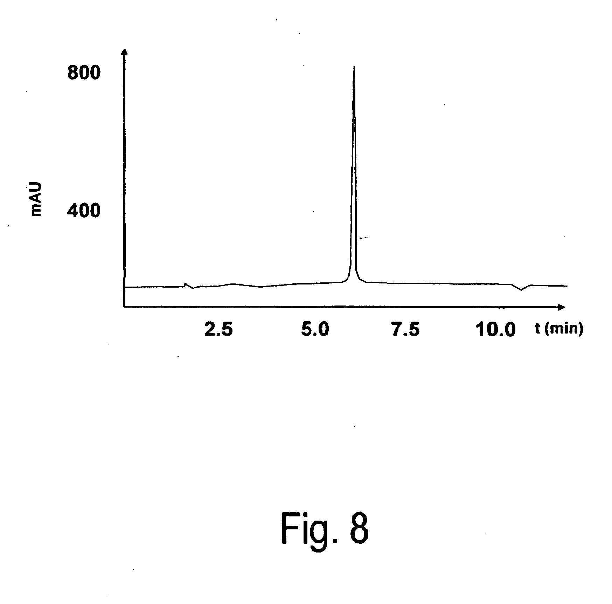

In den Chromatogrammen der Fig. 1 bis Fig. 8 sind auf der γ-Achse die Absorption [mAU = Milliabsorptionseinheit] und auf der x-Achse die Zeit [Minuten] aufgetragen.

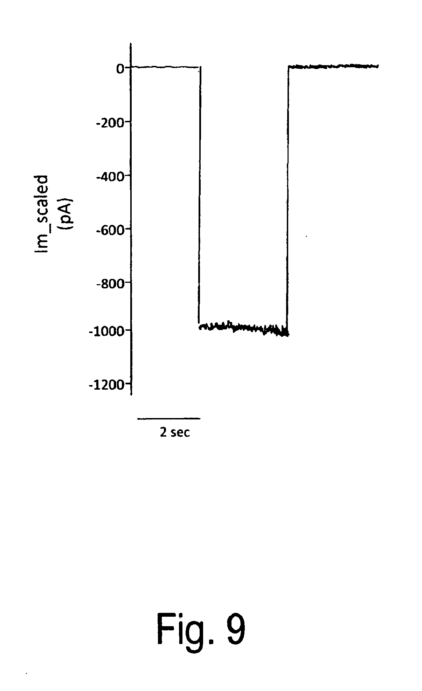

Fig. 9 zeigt das Chromatogramm der Patch Clamp Messung einer Verbindung mit der Aminosäuresequenz SEQ ID NO: 1.

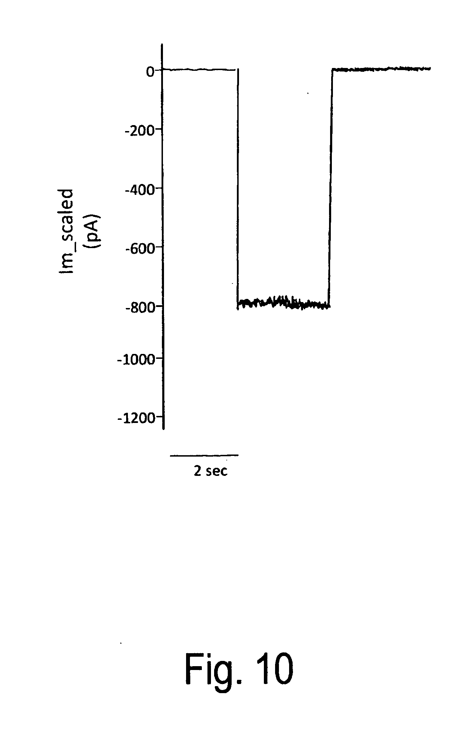

Fig. 10 zeigt das Chromatogramm der Patch Clamp Messung einer Verbindung mit der Aminosäuresequenz SEQ ID NO: 2.

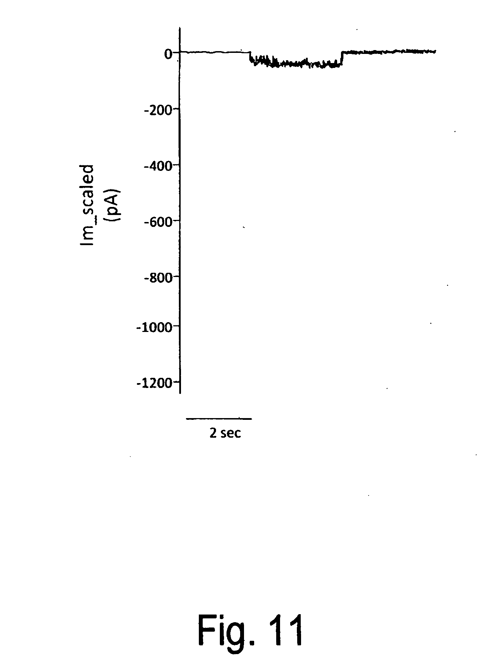

Fig. 11 zeigt das Chromatogramm der Patch Clamp Messung einer Verbindung mit der Aminosäuresequenz SEQ ID NO: 3.

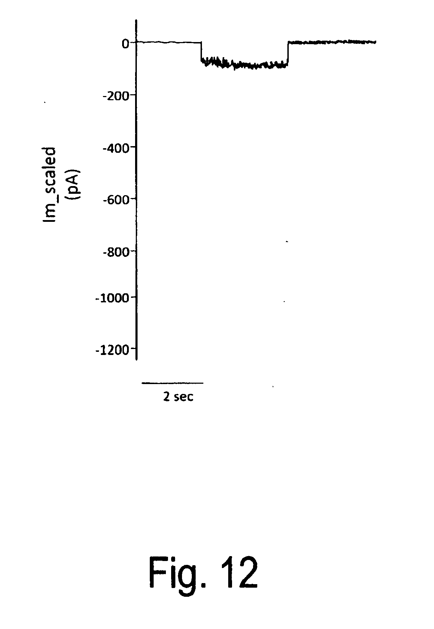

Fig. 12 zeigt das Chromatogramm der Patch Clamp Messung einer Verbindung mit der Aminosäuresequenz SEQ ID NO: 4.

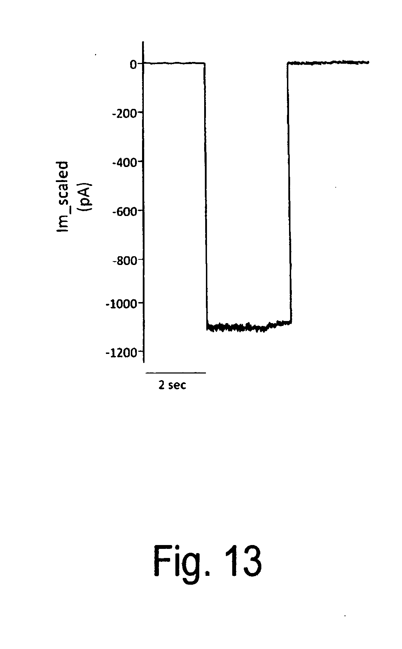

Fig. 13 zeigt das Chromatogramm der Patch Clamp Messung einer Verbindung mit der Aminosäuresequenz SEQ ID NO: 5.

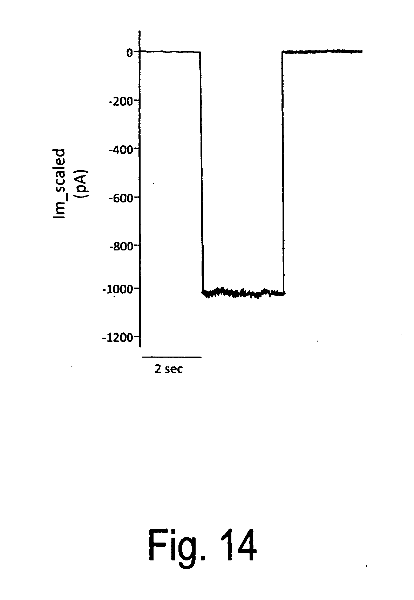

Fig. 14 zeigt das Chromatogramm der Patch Clamp Messung einer Verbindung mit der Aminosäuresequenz SEQ ID NO: 6.

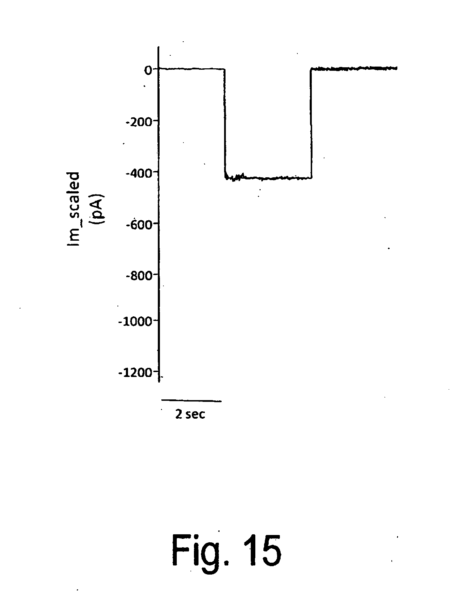

Fig. 15 zeigt das Chromatogramm der Patch Clamp Messung einer Verbindung mit der Aminosäuresequenz SEQ ID NO: 7.

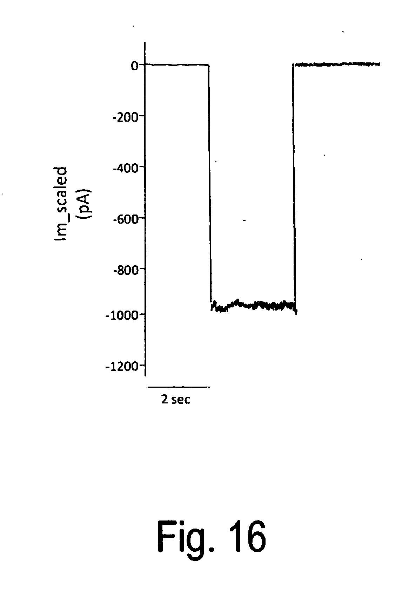

Fig. 16 zeigt das Chromatogramm der Patch Clamp Messung einer Verbindung mit der Aminosäuresequenz SEQ ID NO: 8.

In den Chrömatogrammen der Fig. 9 bis Fig. 16 sind auf der y-Achse die Stromstärke [pA = Picoampere] und auf der x-Achse die Zeit [sec = Sekunden] aufgetragen.

### Beispiel 1

### Synthese einer Verbindung mit der Aminosäuresequenz SEQ ID NO: 1

Die Verbindung mit der Aminosäuresequenz SEQ ID NO: 1 wurde mittels Fmoc-Festphasensynthese vollautomatisch in den Schritten, die in der Tabelle 1 beschrieben sind, synthetisiert:

**Tabelle 1**

| Schritt | Prozess | Produkt |
|---|---|---|
| 1 | sequenzielle Kopplung der Aminosäuren | Wachsende Peptidkette, gebunden an die Festphase |
| 2 | selektive Abspaltung von der Festphase | Teilgeschütztes Peptid in Lösung |
| 3 | Reinigung und Lyophilisation | Gereinigtes, teilgeschütztes Peptid |
| 4 | selektive Zyklisierung | Teilgeschütztes, zyklisiertes Peptid |
| 5 | Abspaltung der Schutzgruppen | Zyklisiertes Peptid in Lösung |
| 6 | Reinigung und Lyophilisation | Gereinigtes, zyklisiertes Peptid als Trifluoressigsäure-Salz |
| 7 | Analytische Untersuchung | Gereinigtes Peptid |

Der Ringschluss erfolgte durch Bildung einer Amidbindung zwischen dem Stickstoff der epsilon-Aminogruppe der Seitenkette des N-terminalen Lysin und dem Kohlenstoff der Karboxylgruppe des C-terminalen Glycin.

Anschließend wurde das Peptid mittels Reverser HPLC untersucht. Die Reinheit betrug mehr als 95%. Das Molekulargewicht betrug 1886,1.

### Beispiel 2

### Synthese einer Verbindung mit der Aminosäuresequenz SEQ ID NO: 2

Die Verbindung mit der Aminosäuresequenz SEQ ID NO: 2 wurde mittels Fmoc-Festphasensynthese vollautomatisch in Schritten, die in der Tabelle 1 im Beispiel 1 beschrieben sind, synthetisiert.

Der Ringschluss erfolgte durch Bildung einer Amidbindung zwischen dem Stickstoff der delta-Aminogruppe der Seitenkette des N-terminalen Ornithins und dem Kohlenstoff der Karboxylgruppe des C-terminalen Glycin.

Anschließend wurde das Peptid mittels Reverser HPLC untersucht. Die Reinheit betrug mehr als 95%. Das Molekulargewicht betrug 1872,4.

### Beispiel 3

### Synthese einer Verbindung mit der Aminosäuresequenz SEQ ID NO: 3

Die Verbindung mit der Aminosäuresequenz SEQ ID NO: 3 wurde mittels Fmoc-Festphasensynthese vollautomatisch in Schritten, die in der Tabelle 1 im Beispiel 1 beschrieben sind, synthetisiert.

Der Ringschluss erfolgte durch Bildung einer Amidbindung zwischen dem Stickstoff der Aminogruppe des N-terminalen Glycin und dem Kohlenstoff C1 der Karboxylgruppe der Amino-Pentansäure einerseits, und durch Bildung einer Amidbindung zwischen dem Stickstoff der Aminogruppe der Amino-Pentansäure und dem Kohlenstoff der Karboxylgruppe des C-terminalen Glycin andererseits.

Anschließend wurde das Peptid mittels Reverser HPLC untersucht. Die Reinheit betrug mehr als 95%. Das Molekulargewicht betrug 1857,0.

### Beispiel 4

### Synthese einer Verbindung mit der Aminosäuresequenz SEQ ID NO: 4

Die Verbindung mit der Aminosäuresequenz SEQ ID NO: 4 wurde mittels Fmoc-Festphasensynthese vollautomatisch in Schritten, die in der Tabelle 1 im Beispiel 1 beschrieben sind, synthetisiert.

Der Ringschluss erfolgte durch Bildung einer Amidbindung zwischen dem Stickstoff der Aminogruppe des N-terminalen Glycin und dem Kohlenstoff C1 der Karboxylgruppe der gamma-Aminobuttersäure einerseits, und durch Herausbildung einer Amidbindung zwischen dem Stickstoff der Aminogruppe der gamma-Aminobuttersäure und dem Kohlenstoff der Karboxylgruppe des C-terminalen Glycin andererseits.

Anschließend wurde das Peptid mittels Reverser HPLC untersucht. Die Reinheit betrug mehr als 95%. Das Molekulargewicht betrug 1843,0.

### Beispiel 5

### Synthese einer Verbindung mit der Aminosäuresequenz SEQ ID NO: 5

Die Verbindung mit der Aminosäuresequenz SEQ ID NO: 5 wurde mittels Fmoc-Festphasensynthese vollautomatisch in Schritten, die in der Tabelle 1 im Beispiel 1 beschrieben sind, synthetisiert.

Der Ringschluss erfolgte durch Bildung einer Amidbindung zwischen dem Stickstoff der Aminogruppe des N-terminalen Glycin und dem Kohlenstoff C1 der Karboxylgruppe der gamma-Aminobuttersäure einerseits, und durch Herausbildung einer Amidbindung zwischen dem Stickstoff der Aminogruppe der gamma-Aminobuttersäure und dem Kohlenstoff der Karboxylgruppe der Seitenkette der C-terminalen Asparaginsäure andererseits.

Anschließend wurde das Peptid mittels Reverser HPLC untersucht. Die Reinheit betrug mehr als 95. Das Molekulargewicht betrug 1901,0.

### Beispiel 6

### Synthese einer Verbindung mit der Aminosäuresequenz SEQ ID NO: 6

Die Verbindung mit der Aminosäuresequenz SEQ ID NO: 6 wurde mittels Fmoc-Festphasensynthese vollautomatisch in Schritten, die in der Tabelle 1 im Beispiel 1 beschrieben sind, synthetisiert.

Der Ringschluss erfolgte durch Bildung einer Amidbindung zwischen dem Stickstoff der Aminogruppe des N-terminalen Glycin und dem Kohlenstoff C1 der Karboxylgruppe der 3-amino-Propansäure einerseits, und durch Bildung einer Amidbindung zwischen dem Stickstoff der Aminogruppe der 3-Amino-Propansäure und dem Kohlenstoff der Karboxylgruppe der Seitenkette der C-terminalen Glutaminsäure andererseits.

Anschließend wurde das Peptid mittels Reverser HPLC untersucht. Die Reinheit betrug mehr als 95. Das Molekulargewicht betrug 1901,0.

### Beispiel 7

### Synthese einer Verbindung mit der Aminosäuresequenz SEQ ID NO: 7

Die Verbindung mit der Aminosäuresequenz SEQ ID NO: 7 wurde mittels Fmoc-Festphasensynthese vollautomatisch in Schritten, die in der Tabelle 1 im Beispiel 1 beschrieben sind, synthetisiert.

Der Ringschluss erfolgte durch Bildung einer Amidbindung zwischen dem Stickstoff der Aminogruppe des N-terminalen Glycin und dem Kohlenstoff C1 der Karboxylgruppe der 7-Amino-Heptansäure einerseits, und durch Bildung einer Amidbindung zwischen dem Stickstoff der Aminogruppe der 7-Amino-Heptansäure und dem Kohlenstoff der Karboxylgruppe des C-terminalen Tyrosin andererseits.

Anschließend wurde das Peptid mittels Reverser HPLC untersucht. Die Reinheit betrug mehr als 95%. Das Molekulargewicht betrug 1828,0.

### Beispiel 8

### Synthese einer Verbindung mit der Aminosäuresequenz SEQ ID NO: 8

Die Verbindung mit der Aminosäuresequenz SEQ ID NO: 8 wurde mittels Fmoc-Festphasensynthese vollautomatisch in Schritten, die in der Tabelle 1 im Beispiel 1 beschrieben sind, synthetisiert.

Der Ringschluss erfolgte durch Bildung einer Amidbindung zwischen dem Stickstoff der Aminogruppe des N-terminalen Glycin und dem Kohlenstoff C1 der Karboxylgruppe der 6-Amino-Hexansäure einerseits, und durch Bildung einer Amidbindung zwischen dem Stickstoff der Aminogruppe der 6-Amino-Hexansäure und dem Kohlenstoff der Karboxylgruppe des C-terminalen Glycin andererseits.

Anschließend wurde das Peptid mittels Reverser HPLC untersucht. Die Reinheit betrug mehr als 95%. Das Molekulargewicht betrug 1873,0.

### Beispiel 9

### Patch Clamp Experimente

### 9a. Zellkultur

Die elektrophysiologischen Experimente wurden an humanen A549 Zellen (ATTC Nr. CCL-185) durchgeführt. A549 Zellen sind humane Lungenepithelzellen, die an der Diffusion von Wasser und Elektrolyten in der Lunge beteiligt sind. Die Zellen wurden in RPMI-1640 Medium (Sigma-Aldrich, Produktnummer R6504) mit 1 % Penicillin-Streptomycin und 10% fötalem Kälberserum suspendiert, in Plastikzellkulturgefäße überführt und im Inkubator bei 95% Luft und 5% CO2 bei 37°C kultiviert. Das Medium wurde 2- bis 3-mal in der Woche gewechselt werden. Die Zellen verdoppeln sich in ca. 22 Stunden und eine Zellkonzentration von über 7 x 10⁴ Zellen pro cm² wurde nicht überschritten.

### 9b. Zugabe der Verbindungen

Die Zellen wurden durch Mikroskopie beobachtet. Dabei wurde festgestellt, dass auch die jeweilige Zugabe einer Verbindung mit der Aminosäuresequenz SEQ ID NO: 1, SEQ ID NO: 2, SEQ ID NO: 3, SEQ ID NO: 4, SEQ ID NO: 5, SEQ ID NO: 6, SEQ ID NO: 7 oder SEQ ID NO: 8 keinerlei Änderungen der Morphologie oder des Zellwachstums ergab und es kam durch die jeweilige Zugabe zu keinem Absterben von Zellen.

### 9c. Patch Clamp Experimente

Für die Patch Clamp Experimente wurden die Zellen auf Glasplättchen übertragen.

### 9d. Patch Clamp Messungen

Makroskopische Ströme wurden von A549 Zellen in der "whole cell" Konfiguration der "Patch-clamp" Technik (Hamill et al, Pflugers Arch. 1981, 391(2):85-100., 1981) abgeleitet. Für die Stromableitungen in der "whole cell" Konfiguration wurden folgende Bad- und Elektrodenlösungen verwendet:
Badlösung: 135 mM Natrium Methansulfonat, 10 mM NaCl, 2.7 mM KCl, 1.8 mM CaCl2, 2 mM MgCl2, 5.5 mM Glucose, und 10 mM HEPES, pH 7.4.
Elektrodenlösung: 120 mM Kaliummethansulfonat, 15 mM KCl, 6 mM NaCl, 1 mM Mg2ATP, 2 mM Na3ATP, 10 mM HEPES, and 0.5 mM EGTA (pH 7.2).

Deckgläser mit den darauf kultivierten Zellen wurden in ein 1 ml fassendes Versuchsbad transferiert, auf dem Mikroskoptisch (Axiovert 100, 400-fache Vergrößerung) fixiert und die Zellen mit der oben beschriebenen Badlösung superfundiert. Sodann wurde von einer geeigneten Zelle (welche am Deckglas haftet) der Strom abgeleitet. Dazu wurde eine mit einer Elektrolytlösung gefüllte Mikroelektrode (Glaskapillare mit einer definierten, Hitzepolierten Spitzenöffnung von ca. 1-3 µm, entspricht einen Widerstand der Elektrodenspitze von 3-5 Ω) auf die Zelle aufgesetzt und die Membran angesaugt, sodass ein "Gigaohm-Seal" zwischen Membran und Elektrode gebildet wurde, um den Leckstrom zu minimieren. Bei der "whole cell"-Konfiguration wurde die Membran unter der Elektrodenspitze durchbrochen, damit der Strom, der durch alle Ionenkanäle der Zelle fließt, gemessen werden kann. Bei Erhalt eines Gigaohm-Seals wurde über einen Vorverstärker (CV-4 Headstage, Axon Instruments) und Verstärker (Axopatch 1D, Axon Instr.) ein definiertes Membranhaltepotential angelegt und der Strom, der dabei durch die Ionenkanäle fließt, gemessen.

Das Pulsprotokoll bestand aus einer Hyperpolarisation der Zellmembran auf-100 mV für 5 s und darauf folgender, schrittweiser Depolarisation in 20 mV Schritten auf +100 mV.

Dieses Protokoll wurde vor (Kontrolle) und nach Zugabe von ringförmigen organischen Molekülen durchgeführt. Die so erhaltenen Stromableitungen wurden gespeichert und mit Hilfe des Programms PCLAMP 6.0 analysiert. Dazu wurden die unter Anwesenheit von Amilorid erhaltenen Stromableitungen von den vorher registrierten Strömen subtrahiert, sodass der Amilorid-sensitive Natriumstrom durch die epithelialen Natriumkanäle ermittelt werden konnte.

### 9d. Resultate

### Regulation von Natrium Ionenkanälen durch Verbindungen gemäß vorliegender Erfindung

Die erfindungsgemäßen Verbindungen wurden mittels Patch Clamp Messung auf deren Fähigkeit, vektorielle lonenkanäle zu regulieren untersucht. Dabei zeigte sich dass erfindungsgemäße Verbindungen die Fähigkeit besitzen, vektorielle Ionenkanäle zu regulieren.

Außerdem wurden erfindungsgemäße Verbindungen mit aus der Literatur bekannten Peptiden verglichen und deren Aktivität im Vergleich zu bekannten Peptiden bestimmt.

Die Resultate sind in Tabelle 2 zusammengefasst:

**Tabelle 2**

| Identifikation | Struktur | Aktivität im Vergleich zum Peptid |
|---|---|---|
| | | CGQRETPEGAEKPWYC |
| | | ("TIP-Peptid", Lucas et al. Science 1994 auch WO00/09149) |
| | | |
| SEQ ID NO: 2 ausEP 2009 023 | CGTKPIELGPDEPKAVC | 80% |
| SEQ ID NO: 2 aus PCT AT2008 448 | LSPGQRETPEGAEAKPWYE | 60% |
| SEQ ID NO: 1 gemäß vorliegender Erfindung | [KGQRETPEGAEAKPWYG] (cyclo Kepsilon1-G17) | 150% |
| SEQ ID NO: 2 gemäß vorliegender Erfindung | [Ornithin-GQRETPEGAEAKPWYG] (cyclo Orn-delta1-G17) | 115% |
| SEQ ID NO: 5 gemäß vorliegender Erfindung | [gamma-Aminobuttersäure-GQRETPEGAEAKPWYD-OH] (cyclo 1- Dγ17) | 160% |
| SEQ ID NO: 6 gemäß vorliegender Erfindung | [3-Amino-Propansäure-GQRETPEGAEAKPWYE-OH] | 150% |
| SEQ ID NO: 8 gemäß vorliegender Erfindung | [6-Amino-Hexansäure-GQRETPEGAEAKPWYG] (cyclo 1-17) | 150% |

Wie aus der Tabelle 2 ersichtlich, ist die Aktivität von Verbindungen gemäß vorliegender Erfindung überraschenderweise höher, als die Aktivität von strukturell verschiednen, bekannten Peptidverbindungen.

## Patentansprüche

1. Zyklische organische Verbindung, die die Aminosäuresequenz GQRETPEGAEAKPWY neben einer oder zwei weiteren Aminosäuren umfasst, **dadurch gekennzeichnet, dass** sie C-terminal über keine Karboxylgruppe und/oder N-terminal über keine Aminogruppe verfügt, wobei eine der Aminosäuren eine nicht natürliche Aminosäure ist, ausgewählt aus Ornithin oder einer omega-Aminosäure, und wobei der Ringschluss zwischen einer Seitenkette einer Aminosäure mit dem C-Terminus einer anderen Aminosäure ausgebildet ist, oder der Ringschluss ist erfolgt mit Hilfe einer nicht natürlichen Aminosäure; oder
die zyklische organische Verbindung ist eine Verbindung der SEQ ID NO: 1

2. Zyklische organische Verbindung nach Anspruch 1, **dadurch gekennzeichnet, dass** die omega-Aminosäure eine omega-Amino-(C₃₋₁₂)Alkansäure ist.

3. Zyklische organische Verbindung nach einem der Ansprüche 1 oder 2, **dadurch gekennzeichnet, dass** die omega-Aminosäure ausgewählt ist aus 3-Amino-Propansäure, gamma-Aminobuttersäure, 5-Amino-Pentansäure, 6-Amino-Hexansäure und 7-Amino-Heptansäure.

4. Zyklische organische Verbindung nach einem der Ansprüche 1 bis 3, **dadurch gekennzeichnet, dass** die nicht natürliche Aminosäure über Amidbindungen verknüpft ist.

5. Zyklische organische Verbindung gemäß Anspruch 1, **dadurch gekennzeichnet, dass** der Ringschluss zwischen einer Seitenkette des Ornithins mit dem C-Terminus einer natürlichen Aminosäure, insbesondere Glycin, ausgebildet ist.

6. Zyklische organische Verbindung gemäß Anspruch 1, worin die Verbindung eine Verbindung der Formel oder ist.

7. Verbindung nach einem der Ansprüche 1 bis 6 in der Form eines Salzes.

8. Verbindung nach einem der Ansprüche 1 bis 7 zur Verwendung als ein Medikament.

9. Verbindung nach einem der Ansprüche 1 bis 7, **dadurch gekennzeichnet, dass** die Verbindung zur Regulierung von vektoriellen Ionenkanälen, insbesondere von Ionenkanälen der Lunge, zur Behandlung von Krankheiten, die mit der Lungenfunktion assoziiert sind, insbesondere zur Behandlung der Akuten Lungen Verletzung (englisch Acute Lung Injury, ALI), des Akuten Respiratorischen Distress Syndrom (englisch Acute Respiratory Distress Syndrome, ARDS), des schweren akuten Atemnotsyndroms (SARS), der Pneumonie, viraler Lungenentzündungen, wie Influenza- und RSV-Infektionen, bei Multiorganversagen, bei Beatmungs-induzierten Lungenschäden, bei Lungentransplantationen, bei Transfusion-assoziierten Lungenschäden, bei therapeutischer Gabe von IL-2 oder von Asthma; und zur Behandlung von Ödemen, insbesondere zur Behandlung des Lungenödems, verwendet wird.

10. Pharmazeutische Zubereitung, **dadurch gekennzeichnet, dass** sie eine Verbindung gemäß einem der Ansprüche 1 bis 7 umfasst.

11. Pharmazeutische Zubereitung gemäß Anspruch 10, **dadurch gekennzeichnet, dass** sie die Verbindung gemäß einem der Ansprüche 1 bis 7 in Verbindung mit zumindest einem pharmazeutisch akzeptablen Hilfsstoff umfasst.

12. Pharmazeutische Zubereitung gemäß Anspruch 11, **dadurch gekennzeichnet, dass** der pharmazeutisch akzeptable Hilfsstoff Träger oder Verdünnungsmittel umfasst.

13. Pharmazeutische Zubereitung gemäß einem der Ansprüche 11 oder 12, **dadurch gekennzeichnet, dass** sie eine Verbindung gemäß einem der Ansprüche 1 bis 7 in Verbindung mit einem oder mehreren Füllstoffen, Bindemitteln, Sprengmitteln, Verbesserern der Fließeigenschaften, Gleitmitteln, Geschmacksstoffen, Zuckern oder Süßstoffen, Geruchsstoffen, Konservierungsstoffen, stabilisierend wirkenden Stoffen, Netzmitteln, Emulgatoren, Lösungsvermittlern, Salzen zur Regulierung des osmotischen Drucks und/oder Puffer(mischungen) umfasst.

## Claims

1. Cyclic organic compound comprising the amino acid sequence GQRETPEGAEAKPWY beside one ort wo further amino acids, **characterized in, that** it does not contain C-terminally a carboxy group and / or N-terminally an amine group, whereby one of the amino acids is an unnatural amino acid, selected from omithin or an omega amino acid, and whereby the ring closure is effected between a side chain of an amino acid with the C-terminus of another amino acid, or the ring closure is effected with the aid of an unnatural amino acid, or
the cyclic organic compound is a compound of SEQ ID NO: 1

2. Cyclic organic compound according to claim 1, **characterized in, that** the omega amino acid is an omega-amino-(C₃₋₁₂)alkanoic acid.

3. Cyclic organic compound according to any one of claims 1 or 2, **characterized in, that** the omega amino acid is selected from 3-amino-propanoic acid, gamma-amino butyric acid, 5-amino-pentanoic acid, 6-amino-hexanoic acid and 7-amino-heptanoic acid.

4. Cyclic organic compound according to any one of claims 1 to 3, **characterized in, that** the unnatural amino acid is bound via amide bonds.

5. Cyclic organic compound according to claim 1, **characterized in, that** the ring closure is effected between the side chain of the ornithin with the C-terminus of a natural amino acid, in particular glycine.

6. Cyclic organic compound according to claim 1, wherein the compound is a compound of formula or

7. Compound according to any one of claims 1 to 6 in the form of a salt.

8. Compound according to any one of claims 1 to 7 for use as a pharmaceutical.

9. Compound according to any one of claims 1 to 7, **characterized in that** the compound is used for the regulation of vectorial ion channels, in particular ion channels of the lung, für the treatment of diseases associated with the lung function, in particular for the treatment of
the acute lung injury (ALI), the acute respiratory distress syndrom (ARDS), the severe acute respiratory syndrome (SARS), the pneumonia, viral lung inflammations, such as influenza- and RSV-infections,
in case of
multi organ dysfunction syndrome, ventilator induced lung damages, lung transplantations, transfusion associated lung damages, therapeutic administration of IL-2, or of asthma, and
for the treatmenr of edemas, in particular for the treatment of lung edema.

10. Pharmaceutical composition, **characterized in that** it comprises a compound according to any one of claims 1 to 7.

11. Pharmaceutical composition according to claim 10, **characterized in that** it comprises a compound according to any one of claims 1 to 7 in association with at least one pharmaceutically acceptable excipient.

12. Pharmaceutical composition according to claim 11, **characterized in that** the pharmaceutically acceptable excipient comprises carrier or diluents.

13. Pharmaceutical composition according to any one of claims 11 or 12, **characterized in that** it comprises a compound according to any one of claims 1 to 7 in association with one or more fillers, binders, disintegrants, flow conditioners, lubricants, flavourings, sugars or sweeteners, odorants, preservatives, stabilizers, wetting agents, emulsifiers, solubilizers, salts for regulating osmotic pressure and/or buffer(mixture)s.

## Revendications

1. Composé organique cyclique, qui comprend la séquence d'acides aminés GQRETPEGAEAKPWY à côté d'un ou de deux autres acides aminés, **caractérisé en ce qu'**il ne dispose en position C-terminale d'aucun groupe carboxyle et/ou en position N-terminale d'aucun groupe amine, l'un des acides aminés étant un acide aminé non naturel choisi parmi l'ornithine ou un acide oméga aminé, et la cyclisation étant formée entre une chaîne latérale d'un acide aminé et le l'extrémité C-terminale d'un autre acide aminé, ou la cyclisation étant réalisée à l'aide d'un acide aminé non naturel ; ou
le composé organique cyclique est un composé de la SEQ ID N° : 1

2. Composé organique cyclique selon la revendication 1, **caractérisé en ce que** l'acide oméga aminé est une acide oméga aminé alcane en C₃₋₁₂.

3. Composé organique cyclique selon l'une des revendications 1 ou 2, **caractérisé en ce que** l'acide oméga aminé est choisi parmi l'acide 3-amino-propanoïque, l'acide gamma-amino butyrique, l'acide 5-amino pentanoïque, l'acide 6-amino hexanoïque et l'acide 7-amino heptanoïque.

4. Composé organique cyclique selon l'une des revendications 1 à 3, **caractérisé en ce que** l'acide aminé non naturel est relié par l'intermédiaire de liaisons amidiques.

5. Composé organique cyclique selon la revendication 1, **caractérisé en ce que** la cyclisation est formée entre une chaîne latérale de l'ornithine avec l'extrémité C-terminale d'un acide aminé naturel, en particulier, la glycine.

6. Composé organique cyclique selon la revendication 1, où le composé est un composé de la formule de la

7. Composé selon l'une des revendications 1 à 6 sous forme d'un sel.

8. Composé selon l'une des revendications 1 à 7 destiné à une utilisation en tant que médicament.

9. Composé selon l'une des revendications 1 à 7, **caractérisé en ce que** le composé est utilisé pour la régulation de canaux ioniques vectoriels, notamment de canaux ioniques des poumons, pour le traitement de maladies qui sont associées à la fonction pulmonaire, en particulier, pour le traitement d'une atteinte pulmonaire aiguë (en Anglais : Acute Lung Injury, ALI), du syndrome de détresse respiratoire aiguë (en Anglais, Acute Respiratory Distress Syndrom, ARDS), du syndrome de détresse respiratoire sévère (SARS), de la pneumonie, de maladies inflammatoires pulmonaires virales, comme l'influenza et les infections RSV, pour des défaillances multi organiques, pour des lésions pulmonaires induites par la ventilation, pour des transplantations de poumon, pour des lésions pulmonaires associées à la transfusion, lors de l'administration d'IL-2 ou pour de l'asthme ; et pour le traitement des oedèmes, en particulier, pour le traitement de l'oedème pulmonaire.

10. Préparation pharmaceutique, **caractérisée en ce qu'**elle comprend un composé selon l'une des revendications 1 à 7.

11. Préparation pharmaceutique selon la revendication 10, **caractérisée en ce que** le composé selon l'une des revendications 1 à 7 comprend au moins un produit auxiliaire pharmaceutiquement acceptable en liaison avec le composé.

12. Préparation pharmaceutique selon la revendication 11, **caractérisée en ce que** le produit auxiliaire acceptable pharmaceutiquement comporte des vecteurs ou des diluants.

13. Préparation pharmaceutique selon l'une des revendications 11 ou 12, **caractérisée en ce que** le composé selon l'une des revendications 1 à 7 comprend, en liaison avec une ou plusieurs charges, des liants, des désintégrants, des améliorants des propriétés d'écoulement, des lubrifiants, des arômes, des sucres ou des édulcorants, des parfums, des sucres ou des édulcorants, des arômes, des agents de conservation, des produits agissant comme stabilisants, les agents mouillants, des émulgateurs, des agents complexants, des sels pour la régulation de la pression osmotique et/ou des (mélanges) tampons.
